(19) Europäisches Patentamt
European Patent Office
Office européen des brevets

(11) **EP 4 309 609 A1**

(12) **EUROPEAN PATENT APPLICATION**
published in accordance with Art. 153(4) EPC

(43) Date of publication:
**24.01.2024 Bulletin 2024/04**

(21) Application number: **22770567.0**

(22) Date of filing: **16.03.2022**

(51) International Patent Classification (IPC):
***A61B 34/20*** *(2016.01)*

(86) International application number:
**PCT/CN2022/081214**

(87) International publication number:
**WO 2022/194214 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.03.2021 CN 202110292417**

(71) Applicant: **Sceneray Co., Ltd.
Jiangsu 215123 (CN)**

(72) Inventors:
• **CHEN, Lei**
  **Suzhou, Jiangsu 215123 (CN)**
• **ZHU, Weiran**
  **Suzhou, Jiangsu 215123 (CN)**

(74) Representative: **Cabinet Beaumont
4, Place Robert Schuman
B.P. 1529
38025 Grenoble Cedex 1 (FR)**

(54) **SYSTEM FOR PRE-OPERATIVELY SCREENING NERVE STIMULATOR IMPLANTATION POSITION IN SKULL**

(57) Provided is a system for pre-operatively screening an implantation position of a nerve stimulator in a skull. The system includes an acquisition module, a reconstruction module, an adaptation calculation module, and an implantation position determination module. The acquisition module is configured to acquire image data of a skull of a patient and a 3D appearance model of the nerve stimulator. The reconstruction module is configured to reconstruct a 3D structure of the skull of the patient according to the image data. The adaptation calculation module is configured to use the 3D appearance model of the nerve stimulator and the 3D structure of the skull of the patient to calculate an adaptation coefficient of a region of interest in which the nerve stimulator is to be implanted in the skull. The implantation position determination module is configured to determine, according to the adaptation coefficient, an optimal position at which the nerve stimulator is to be implanted in the skull.

```
┌─────────────────────────────────┐ 10
│      Acquisition module         │
└─────────────────────────────────┘
               │
               ▼
┌─────────────────────────────────┐ 20
│     Reconstruction module       │
└─────────────────────────────────┘
               │
               ▼
┌─────────────────────────────────┐ 30
│  Adaptation calculation module  │
└─────────────────────────────────┘
               │
               ▼
┌─────────────────────────────────┐ 40
│   Draft implantation position   │
│     determination module        │
└─────────────────────────────────┘
```

**FIG. 2**

EP 4 309 609 A1

**Description**

The present application claims priority to Chinese Patent Application No. 202110292417.X filed with the China National Intellectual Property Administration (CNIPA) on Mar. 18, 2021, the disclosure of which is incorporated herein by reference in its entirety.

TECHNICAL FIELD

The present application relates to the field of medical equipment technology, for example, a system for pre-operatively screening an implantation position of a nerve stimulator in a skull.

BACKGROUND

A nerve stimulator system implanted in a skull consists of a nerve stimulator implanted in the skull and a stimulation electrode implanted in a deep brain and is configured to treat nervous system disorders such as epilepsy. Due to a certain thickness, length, width and volume of the nerve stimulator implanted in the skull and due to individual differences among skulls, it is one of the key issues in this kind of surgery how an appropriate implantation position is selected to implement the optimal adaptation of the stimulator to the radian and thickness of the skull, so as to guarantee the safety after implantation to the maximum extent and reduce side effects after the implantation as much as possible.

Considering factors like the radian of the skull at the implantation position and the range of removed bones, the nerve stimulator is designed with a shape of a certain radian to implement the optimal adaptation of the nerve stimulator to the skull, after being implanted in the skull. Clinically, the common implantation positions of a Neurospace's nerve stimulator implanted in the skull are the parietal bone and the occipital bone. Typically, a neurosurgeon usually compares the skull shape of a patient with the shape of the nerve stimulator in surgery and performs a craniectomy after selecting an approximate range. The manner in which the skull is adapted to the shape of the nerve stimulator while the skull is removed may prolong surgery duration. More importantly, due to individual differences, such an adaptation manner of a nerve stimulator cannot be implemented in a practical application of clinical surgery, which has the manifestation that one part of the nerve stimulator is well adapted to the skull while the other part is upturned and protrudes from the skull surface. The neurosurgeon may compromise finally and select one implantation position, radian and angle for fixation. In this case, complications may easily occur clinically. For example, after the skin is sutured, the head of the patient has an abnormal appearance, which affects aesthetics; or the sutured skin has excessive tension, leading to a poor blood supply of the local skin, affecting wound healing, and even making nerve stimulator stay exposed after implantation for a period of time. Additionally, the implantation of the poorly-adapted nerve stimulator may also affect the body position of the patient during sleep, and the head of the patient fails to be tilted to the implantation side.

SUMMARY

The present application provides a system for pre-operatively screening an implantation position of a nerve stimulator in a skull. The system includes an acquisition module, a reconstruction module, an adaptation calculation module, and a draft implantation position determination module.

The acquisition module is configured to acquire image data of a skull of a patient and a three-dimension (3D) appearance model of the nerve stimulator.

The reconstruction module is configured to reconstruct a 3D structure of the skull of the patient according to the image data.

The adaptation calculation module is configured to calculate, by using the 3D appearance model of the nerve stimulator and the 3D structure of the skull of the patient, an adaptation coefficient of a region of interest in which the nerve stimulator is to be implanted in the skull.

The draft implantation position determination module is configured to determine, according to the adaptation coefficient, a position at which the nerve stimulator is to be implanted in the skull.

BRIEF DESCRIPTION OF DRAWINGS

FIG. 1 is a flowchart of a method for pre-operatively screening an implantation position of a nerve stimulator in a skull according to embodiment one of the present application.

FIG. 2 is a functional block diagram of a system for pre-operatively screening an implantation position of a nerve stimulator in a skull according to embodiment two of the present application.

Reference list

10 acquisition module

20 reconstruction module

30 adaptation calculation module

40 draft implantation position determination module

DETAILED DESCRIPTION

The present application is described hereinafter in detail in conjunction with drawings and embodiments so that those skilled in the art can understand and implement the present application.

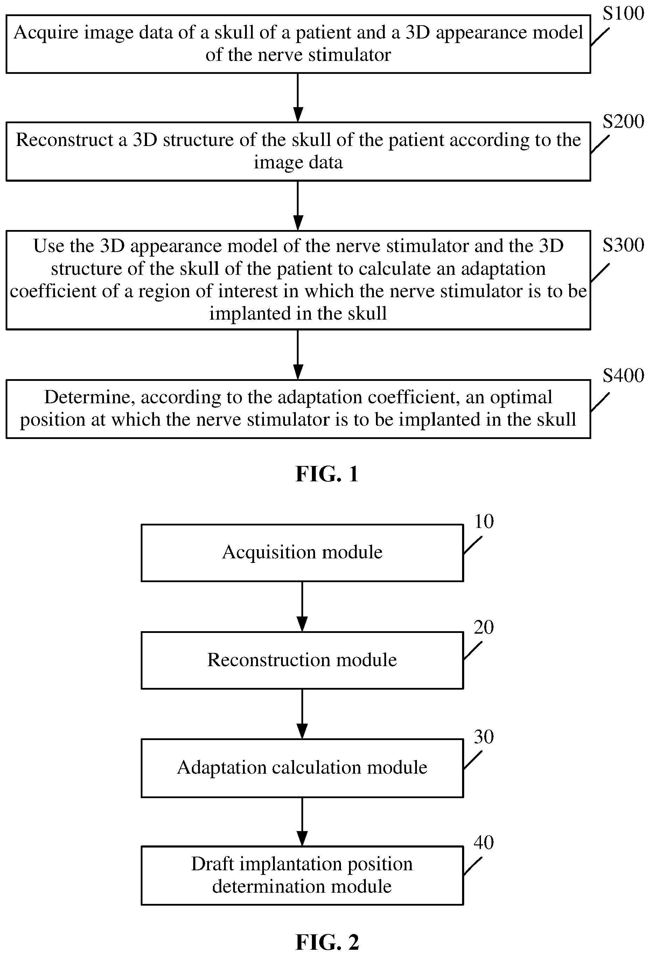

In order to understand a method and system for pre-operatively screening an implantation position of a nerve stimulator in a skull disclosed in embodiments of the present application, the present application provides a flowchart of a method for pre-operatively screening an implantation position of a nerve stimulator in a skull, as shown in FIG. 1.

Referring to FIG. 1, an embodiment of the present application provides a method for pre-operatively screening an implantation position of a nerve stimulator in a skull. The method includes the steps below.

In S 100, image data of a skull of a patient and a three-dimension (3D) appearance model of the nerve stimulator are acquired.

Illustratively, the image data may be computed tomography (CT) data. The CT data of digital imaging and communications in medicine (DICOM) of tissue imaging and skull bone window imaging may be acquired by scanning the skull CT of the patient. The CT data in this embodiment may be engraved on a compact disk (CD) for backup. The image data may also be magnetic resonance imaging (MRI) data, which is not limited in the present application.

Illustratively, the 3D appearance model of the nerve stimulator may be constructed using software. The software may be, for example, CREO. The 3D appearance model of the same nerve stimulator may be backed up for different individuals.

In S200, a 3D structure of the skull of the patient is reconstructed according to the image data.

Illustratively, the image data may be CT data. The CT data of the skull of the patient is imported into software. A threshold is selected in the software to reconstruct the 3D structure of the skull of the patient. For example, the CT data of the skull of the patient is imported into the MimicsMedical software to reconstruct the 3D structure of the skull of the patient for backup. The surgery process in the MimicsMedical software may be as follows: The DICOM data is imported; a skull threshold range is selected; and the 3D structure of the skull is constructed.

In S300, the 3D appearance model of the nerve stimulator and the 3D structure of the skull of the patient are used to calculate an adaptation coefficient of a region of interest in which the nerve stimulator is to be implanted in the skull.

Illustratively, an adaptation calculation model for the to-be-implanted region of interest in the skull and the nerve stimulator is constructed. The adaptation coefficient of the region of interest in which the nerve stimulator is to be implanted in the skull is calculated through the adaptation calculation model. The adaptation calculation model is as follows: $\Phi = T * AP * SI * FA * H * P$. $\Phi$ denotes the adaptation coefficient. T denotes the skull thickness of the region of interest. AP denotes the skull front-rear radian of the region of interest. SI denotes the skull upper-lower radian of the region of interest. FA denotes the importance of tissue below the region of interest in which the nerve stimulator is to be implanted in the skull. H denotes the maximum height of the nerve stimulator protruding from a skull surface after the nerve stimulator is to be implanted in the skull. P denotes the pressure condition of skin above the nerve stimulator in daily life after the nerve stimulator is to be implanted in the skull.

The skull thickness T of the region of interest, the skull front-rear radian AP of the region of interest, and the skull upper-lower radian SI of the region of interest that are in the adaptation calculation model are measured and obtained from the 3D structure of the skull of the patient. The measuring steps belong to the related art and are not repeated here in the present application. When the importance FA of the tissue below the region of interest in which the nerve stimulator is to be implanted in the skull is determined, reference may be made to Brodmann areas. Brain tissue is divided into 52 areas which are scored according to importance. Whether a great vessel exists on the tissue surface is scored separately. The larger the FA score, the more important the tissue below. The smaller the FA score, the less important the tissue below. The maximum height H of the nerve stimulator protruding from the skull surface after the nerve stimulator is to be implanted in the skull is a distance between the highest point of the appearance model and the skull surface after the 3D appearance model of the nerve stimulator is implanted in the skull. The pressure condition P of the skin above the nerve stimulator in daily life after the nerve stimulator is to be implanted in the skull includes whether the skin above is compressed during sleep, the compression range during sleep, whether the skin above is compressed when the patient wears a hat, and the compression range when the patient wears the hat.

Illustratively, the region of interest on the 3D structure of the skull of the patient may be automatically screened. For example, after the 3D appearance model of the nerve stimulator and the 3D structure of the skull of the patient are imported into the adaptation calculation model, the model automatically screens multiple regions of interest corresponding to the position for implantation in the skull. Moreover, the adaptation coefficient of each region of interest is calculated. The region of interest on the 3D structure of the skull of the patient may be manually screened. For example, a medical worker manually draws multiple regions of interest of positions that the medical worker may consider to be used for implantation in the skull, and then the adaptation coefficient of each region of interest is calculated through the adaptation calculation model.

In S400, the optimal position at which the nerve stimulator is to be implanted in the skull is determined according to the adaptation coefficient.

Illustratively, adaptation coefficients of multiple regions of interest are compared. A larger adaptation coefficient indicates that the corresponding region of interest is closer to the optimal position at which the nerve stimulator is to be implanted in the skull.

The method in this embodiment further includes the following step: After the optimal position at which the nerve stimulator is to be implanted in the skull is determined, a region of interest other than the region of interest corresponding to the optimal position on the 3D structure of the skull of the patient is removed, or made transparent, or hidden; and the adaptation calculation model is used to verify the adaptation coefficient of the optimal position at which the nerve stimulator is to be implanted in the skull.

For the method for pre-operatively screening an implantation position of a nerve stimulator in a skull according to this embodiment of the present application, the 3D appearance model of the nerve stimulator and the 3D structure of the skull of the patient are used to calculate the adaptation coefficient of the region of interest in which the nerve stimulator is to be implanted in the skull, and the optimal position at which the nerve stimulator is to be implanted in the skull is determined according to the adaptation coefficient, thereby implementing the pre-operative screening of the implantation position of the nerve stimulator in the skull. On one hand, a doctor can pre-operatively select the individualized optimal implantation position in the skull according to the individual difference of the patient and shorten surgery duration. On the other hand, the adaptation of the implanted nerve stimulator to the skull can be truly demonstrated and evaluated, the individualized adaptation of the nerve stimulator to the skull is truly implemented, the safety after implantation is guaranteed to the maximum extent, side effects after implantation are reduced as much as possible, and the difficulty and complication problem of implanting the nerve stimulator in the skull are solved.

A system for pre-operatively screening an implantation position of a nerve stimulator in a skull according to embodiment two of the present application is introduced hereinafter. The system for pre-operatively screening an implantation position of a nerve stimulator in a skull described hereinafter and the preceding method for pre-operatively screening an implantation position of a nerve stimulator in a skull may be referred to each other correspondingly.

Referring to FIG. 2, an embodiment of the present application provides a system for pre-operatively screening an implantation position of a nerve stimulator in a skull. The system includes an acquisition module 10, a reconstruction module 20, an adaptation calculation module 30, and a draft implantation position determination module 40.

The acquisition module 10 is configured to acquire the image data of a skull of a patient and a 3D appearance model of the nerve stimulator. The reconstruction module 20 is configured to reconstruct a 3D structure of the skull of the patient according to the image data. The adaptation calculation module 30 is configured to calculate, by using the 3D appearance model of the nerve stimulator and the 3D structure of the skull of the patient, an adaptation coefficient of a region of interest in which the nerve stimulator is to be implanted in the skull. The draft implantation position determination module 40 is configured to determine, according to the adaptation coefficient, an optimal position at which the nerve stimulator is to be implanted in the skull.

In an embodiment, the reconstruction module 20 is configured to import the image data of the skull of the patient into software and select a threshold in the software to reconstruct the 3D structure of the skull of the patient.

In an embodiment, the adaptation calculation module 30 is configured to construct an adaptation calculation model for the to-be-implanted region of interest in the skull and the nerve stimulator and calculate, through the adaptation calculation model, the adaptation coefficient of the region of interest in which the nerve stimulator is to be implanted in the skull.

In an embodiment, the adaptation calculation model is as follows: $\Phi = T * AP * SI * FA * H * P$. $\Phi$ denotes the adaptation coefficient. T denotes the skull thickness of the region of interest. AP denotes the skull front-rear radian of the region of interest. SI denotes the skull upper-lower radian of the region of interest. FA denotes the importance of tissue below the region of interest in which the nerve stimulator is to be implanted in the skull. H denotes the maximum height of the nerve stimulator protruding from a skull surface after the nerve stimulator is to be implanted in the skull. P denotes the pressure condition of the skin above the nerve stimulator after the nerve stimulator is to be implanted in the skull.

In an embodiment, the acquisition module 10 is also configured to measure and obtain, from the 3D structure of the skull of the patient, the skull thickness T of the region of interest, the skull front-rear radian AP of the region of interest, and the skull upper-lower radian SI of the region of interest.

In an embodiment, the region of interest on the 3D structure of the skull of the patient is automatically screened.

In an embodiment, the 3D appearance model of the nerve stimulator is constructed using software.

In an embodiment, the system for pre-operatively screening an implantation position of a nerve stimulator in a skull further includes a verification module. The verification module is configured to, after the position at which the nerve stimulator is to be implanted in the skull is determined, remove, make transparent, or hide a region of interest other than the region of interest corresponding to the optimal position on the 3D structure of the skull of the patient and use the adaptation calculation model to verify the adaptation coefficient of the optimal position at which the nerve stimulator is to be implanted in the skull.

Since the system for pre-operatively screening an implantation position of a nerve stimulator in a skull in this embodiment is used for implementing the preceding method for pre-operatively screening an implantation position of a nerve stimulator in a skull, the function of the system corresponds to the function of the preceding method and is not repeated here.

The present application may take the form of an entirely hardware embodiment, an entirely software embodiment or an embodiment combining software and hardware aspects. Moreover, the present application may take the form of a computer program product implemented on one or more computer-usable storage media (including, but not limited to, a disk memory, a compact disc read-only memory (CD-ROM), an optical memory, and the like) that include computer-usable program codes.

The present application is described with reference to flowcharts and/or block diagrams of methods, apparatuses (systems) and computer program products according to the embodiments of the present application. It is to be understood that each flow and/or block in the flowcharts and/or block diagrams and a combination of flows and/or blocks in the flowcharts and/or block diagrams are implemented by computer program instructions. These computer program instructions may be provided to a general-purpose computer, a special-purpose computer, an embedded processor or a processor of other programmable data processing equipment to produce a machine so that instructions executed by a computer or the processor of other programmable data processing equipment produce a means for implementing functions specified in one or more flows in the flowcharts and/or one or more blocks in the block diagrams.

These computer program instructions may also be stored in a computer-readable memory which can direct the computer or other programmable data processing equipment to operate in a particular manner so that the instructions stored in the computer-readable memory produce a manufactured product including an instruction means. The instruction means implements the functions specified in one or more flows in the flowcharts and/or one or more blocks in the block diagrams.

These computer program instructions may also be loaded onto the computer or other programmable data processing equipment so that a series of surgery steps are performed on the computer or other programmable equipment to produce processing implemented by the computer. Therefore, instructions executed on the computer or other programmable equipment provide steps for implementing the functions specified in one or more flows in the flowcharts and/or one or more blocks in the block diagrams.

## Claims

1. A system for pre-operatively screening an implantation position of a nerve stimulator in a skull, comprising:

   an acquisition module configured to acquire image data of a skull of a patient and a three-dimensional, 3D, appearance model of the nerve stimulator;
   a reconstruction module configured to reconstruct a 3D structure of the skull of the patient according to the image data;
   an adaptation calculation module configured to calculate, by using the 3D appearance model of the nerve stimulator and the 3D structure of the skull of the patient, an adaptation coefficient of a region of interest in which the nerve stimulator is to be implanted in the skull; and
   a draft implantation position determination module configured to determine, according to the adaptation coefficient, a position at which the nerve stimulator is to be implanted in the skull.

2. The system according to claim 1, wherein the reconstruction module is configured to:

   import the image data of the skull of the patient into software; and
   select a threshold in the software to reconstruct the 3D structure of the skull of the patient.

3. The system according to claim 1, wherein the adaptation calculation module is configured to:

   construct an adaptation calculation model for the to-be-implanted region of interest in the skull and the nerve stimulator; and
   calculate, through the adaptation calculation model, the adaptation coefficient of the region of interest in which the nerve stimulator is to be implanted in the skull.

4. The system according to claim 3, wherein the adaptation calculation model is as follows:

$$\Phi = T * AP * SI * FA * H * P,$$

   wherein $\Phi$ denotes the adaptation coefficient, T denotes a skull thickness of the region of interest, AP denotes a skull front-rear radian of the region of interest, SI denotes a skull upper-lower radian of the region of interest, FA denotes importance of tissue below the region of interest in which the nerve stimulator is to be implanted in the skull, H denotes a maximum height of the nerve stimulator protruding from a skull surface after the nerve stimulator is to be implanted in the skull, and P denotes pressure condition of skin above the nerve stimulator after the nerve stimulator is to be implanted in the skull.

5. The system according to claim 4, wherein the acquisition module is further configured to:
measure and obtain, from the 3D structure of the skull of the patient, the skull thickness T of the region of interest, the skull front-rear radian AP of the region of interest, and the skull upper-lower radian SI of the region of interest.

6. The system according to claim 1, wherein the region of interest on the 3D structure of the skull of the patient is automatically screened.

7. The system according to claim 1, wherein the region of interest on the 3D structure of the skull of the patient is manually screened.

8. The system according to claim 1, wherein the 3D appearance model of the nerve stimulator is constructed using software.

9. The system according to claim 1, further comprising:
a verification module configured to, after the position at which the nerve stimulator is to be implanted in the skull is determined, remove, make transparent, or hide a region of interest other than the region of interest corresponding to the determined position on the 3D structure of the skull of the patient and use an adaptation calculation model to verify the adaptation coefficient of the determined position at which the nerve stimulator is to be implanted.

Acquire image data of a skull of a patient and a 3D appearance model of the nerve stimulator

S100

Reconstruct a 3D structure of the skull of the patient according to the image data

S200

Use the 3D appearance model of the nerve stimulator and the 3D structure of the skull of the patient to calculate an adaptation coefficient of a region of interest in which the nerve stimulator is to be implanted in the skull

S300

Determine, according to the adaptation coefficient, an optimal position at which the nerve stimulator is to be implanted in the skull

S400

**FIG. 1**

Acquisition module

10

Reconstruction module

20

Adaptation calculation module

30

Draft implantation position determination module

40

**FIG. 2**

## INTERNATIONAL SEARCH REPORT

| International application No. |
| --- |
| **PCT/CN2022/081214** |

**A. CLASSIFICATION OF SUBJECT MATTER**

A61B 34/20(2016.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT, WPABSC, ENTXTC, CNKI: 刺激, 电极, 模型, 颅骨, 头颅, 三维, 植入, 计划, 位置, 选择, 识别, 适配, 系数, 图像, 重建; WPABS, ENTXT, DWPI, VEN: stimulat+, electrode, model, skull, three-dimensional, implant+, plan+, locat+, select+, identify, adapt+, coefficient, image, reconstruct+

**C. DOCUMENTS CONSIDERED TO BE RELEVANT**

| Category* | Citation of document, with indication, where appropriate, of the relevant passages | Relevant to claim No. |
| --- | --- | --- |
| PX | CN 113081259 A (SUZHOU JINGYU MEDICAL EQUIPMENT CO., LTD.) 09 July 2021 (2021-07-09) claims 1-10, and description, paragraphs 35-62, and figures 1-2 | 1-9 |
| X | WO 2011135290 A1 (RENISHAW IRELAND LTD.) 03 November 2011 (2011-11-03) description, page 2, line 15 to page 6, line 30, and figures 1-3 | 1-9 |
| X | CN 111615372 A (MEDTRONIC INC.) 01 September 2020 (2020-09-01) description, paragraphs 30-181, and figures 1-14 | 1-9 |
| A | CN 108294814 A (XUANWU HOSPITAL, CAPITAL MEDICAL UNIVERSITY et al.) 20 July 2018 (2018-07-20) entire document | 1-9 |
| A | CN 109658400 A (BEIJING TIANTAN HOSPITAL, CAPITAL MEDICAL UNIVERSITY) 19 April 2019 (2019-04-19) entire document | 1-9 |
| A | US 2013172731 A1 (GOLE, P. D.) 04 July 2013 (2013-07-04) entire document | 1-9 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **31 May 2022** | **15 June 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/081214** |

**C.      DOCUMENTS CONSIDERED TO BE RELEVANT**

| Category* | Citation of document, with indication, where appropriate, of the relevant passages | Relevant to claim No. |
|---|---|---|
| A | WO 2020043291 A1 (BRAINLAB AG) 05 March 2020 (2020-03-05)<br>entire document | 1-9 |

Form PCT/ISA/210 (second sheet) (January 2015)

**INTERNATIONAL SEARCH REPORT**
**Information on patent family members**

International application No.

**PCT/CN2022/081214**

| Patent document cited in search report | | | Publication date (day/month/year) | Patent family member(s) | | | Publication date (day/month/year) |
|---|---|---|---|---|---|---|---|
| CN | 113081259 | A | 09 July 2021 | | None | | |
| WO | 2011135290 | A1 | 03 November 2011 | GB | 201006971 | D0 | 09 June 2010 |
| CN | 111615372 | A | 01 September 2020 | US | 2019214126 | A1 | 11 July 2019 |
| | | | | EP | 3737320 | A1 | 18 November 2020 |
| | | | | WO | 2019139690 | A1 | 18 July 2019 |
| | | | | US | 2020111560 | A1 | 09 April 2020 |
| | | | | US | 10535427 | B2 | 14 January 2020 |
| CN | 108294814 | A | 20 July 2018 | | None | | |
| CN | 109658400 | A | 19 April 2019 | | None | | |
| US | 2013172731 | A1 | 04 July 2013 | WO | 2013102190 | A1 | 04 July 2013 |
| | | | | EP | 2797512 | A1 | 05 November 2014 |
| | | | | AU | 2012362187 | A1 | 10 July 2014 |
| | | | | CA | 2862369 | A1 | 04 July 2013 |
| WO | 2020043291 | A1 | 05 March 2020 | US | 2021322101 | A1 | 21 October 2021 |
| | | | | EP | 3843651 | A1 | 07 July 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110292417X